# EUROPEAN PATENT SPECIFICATION

(11) **EP 1 202 676 B1**
(45) Date of publication and mention of the grant of the patent: **22.09.2010**
(21) Application number: 00951800.2
(22) Date of filing: 11.08.2000
(51) Int. Cl.: A61B 17/22, A61F 2/01

(54) **RETRIEVAL DEVICE**
MEDIZINISCHE FASSVORRICHTUNG
DISPOSITIF D'EXTRACTION

(30) Priority: 12.08.1999 WO PCT/IE99/00082
(43) Date of publication of application: 08.05.2002
(73) Proprietor: Salviac Limited, Dublin 2 (IE)
(72) Inventor: Vale, David, Clontarf, Dublin 3 (IE); Gilson, Paul, County Galway (IE); Griffin, Patrick, Castlegar, County Galway (IE)
(74) Representative: O'Brien, John Augustine
(86) International application number: PCT/IE2000/000097
(87) International publication number: WO 2001/012082

(56) References cited:
- EP-A- 0 827 756
- WO-A-00/44428
- WO-A-97/03810
- WO-A-97/17021
- WO-A-97/17914
- WO-A-98/30265
- WO-A-98/39053
- WO-A-99/20335
- FR-A- 2 616 666
- US-A- 4 611 594
- US-A- 4 927 426
- US-A- 5 011 488
- US-A- 5 593 394

## Description

The invention relates to a retrieval device for retrieving a medical device from a body lumen. More particularly, the retrieval device is for retrieving an embolic filter device deployed in the vasculature of a patient to provide protection against embolic material dislodged during a surgical or medical procedure. Such an embolic protection device is described in our WO 99/23976A.

To retrieve a deployed embolic filter from a body lumen a retrieval catheter is used. The catheter is in the form of an elongated tube with an open mouth at the distal end into which the deployed embolic filter is retrieved. There is however a problem with such retrieval catheters in that the open mouth can become snagged at sharp changes in direction in the body lumen, at stenosed regions, or on other deployed medical devices such as stents. This is particularly the case with an embolic filter which is deployed downstream of a stenosis during an angioplasty.

There is therefore a need for an improved retrieval device which will overcome this problem.

### Statements of Invention

WO 98/39053A describes an embolic filter retrieval device for retrieving a medical device from a body lumen, through a lesion or a treatment device proximal of the filter.

The retrieval device comprises:-a retrieval catheter including an elongate tube having a proximal end and a distal end, the retrieval catheter defining a retrieval space, into which an embolic filter is at least partially retrieved, at the distal end of the elongate tube;
a centring catheter having a distal end, the centring catheter being mounted in the retrieval catheter for movement between an introduction configuration, for introduction of the retrieval device through a lesion or a treatment device to an embolic filter positioned outside the retrieval space of the retrieval catheter, in which the distal end of the centring catheter projects distally from the distal end of the retrieval catheter, and a retrieval configuration in which the distal end of the centring catheter is proximal of the retrieval space of the retrieval catheter, and the embolic filter is at least partially retrieved into the retrieval space of the retrieval catheter;
a guidewire extending at least in part through the centering catheter, the guidewire having a proximal end and a distal end;
said embolic filter mounted on or engagable with the guidewire proximate the distal end of the guidewire.

The invention is characterised in that the retrieval catheter has a radially expansible tip at the distal end to accommodate retrieval of the embolic filter into the retrieval space. Thus the retrieval catheter may retrieve medical devices with a diameter greater than the relaxed diameter of the catheter. The tip may be thin-walled to ensure a low crossing profile for the retrieval device. The tip has sufficient axial stiffness to assist the retrieval of the embolic filter.

The centring means facilitates the crossing of a lesion or a treatment device such as a stent to facilitate retrieval of an embolic filter distal of the lesion or deployed stent.

According to the invention the centring catheter has an internal bore extending at least partially along its length for threading the retrieval device over a guidewire. The centring catheter positions the guidewire centrally in the body lumen away from the lumen walls.

The centring catheter is slidably movable in the retrieval catheter from the introduction configuration to the retracted retrieval configuration. This provides retrieval space in the retrieval catheter.

Ideally the centring catheter is removable from the retrieval catheter. This allows procedures such as aspiration to be carried out. Other devices, such as a snare, may also be introduced.

In another embodiment of the invention a proximal end of the centring catheter extends proximally from the proximal end of the retrieval catheter for external manipulation of the centring catheter relative to the retrieval catheter.

In a particularly preferred embodiment the distal end of the centring catheter is tapered distally inwardly to guide the open mouth through the body lumpen. Ideally the distal end of the centring catheter is shaped to provide a smooth transition between the distal end of the centring catheter and the distal end of the retrieval catheter. Desirably the distal end of the centring catheter is of arrowhead shape. In this manner the retrieval device has a smooth, step free crossing profile for safe, snag-free advancement through the body lumen.

Preferably the distal end of the centring catheter is flexible to facilitate navigation through the vasculature.

In another embodiment the distal end of the centring catheter is sealably engagable to the distal end of this retrieval catheter.

Herein there is also disclosed a retrieval catheter having a main catheter body and the centring means is a tapered distal extension of the main body of the retrieval catheter, the tapered distal extension having an open mouth through which a medical device is retrieved.

In one embodiment at least portion of the distal extension is movable inwardly between the introduction and retrieval configurations. In this case preferably the distal extension is inverted on movement from the introduction to the retrieval configurations. Preferably the distal extension is of resilient elastomeric material.

In another embodiment at least portion of the distal extension is movable radially outwardly between the introduction and retrieval configurations.

The distal end of the centring catheter may be at least partially radiopaque. This assists in guiding the distal end through the body lumen to the medical device to be retrieved.

The centring catheter may be of or coated with a material with a low coefficient of friction. In this way advancement of the centring means through the body lumen is eased.

In another embodiment of the invention the diameter of the retrieval catheter varies along its length.

In a particularly preferred embodiment the distal end of the retrieval catheter is tapered distally inwardly to provide a smooth crossing profile for the retrieval device. This ensures that the retrieval device has a smooth, step-free crossing profile.

In another preferred embodiment the inner surface of the retrieval catheter and/ or the outer surface of the centring catheter is of non-circular profile over at least portion of the length thereof. Preferably, the catheter periphery is non-circular. Ideally the catheter periphery is of oval shape.

In one embodiment of the invention the catheter periphery is shaped to define a number of separate areas of contact with the other catheter. The catheter periphery may be of fluted shape.

The retrieval device of the invention is particularly adapted for retrieving an embolic filter from a blood vessel.

Herein there is also disclosed a method of retrieving a medical device from a body lumen comprising the steps of: -
introducing a retrieval catheter with a centring means into a body lumen, the retrieval catheter defining a retrieval space, and the centring means having an introduction configuration for introduction of the retrieval catheter and a retrieval configuration for retrieving a medical device;
advancing the retrieval catheter across a lesion or a treatment device with the centring means in the introduction configuration;
advancing the retrieval catheter distally to the proximal end of the medical device;
retrieving the medical device into the retrieval catheter with the centring means in the retrieval configuration; and
removing the retrieval catheter and the retrieved medical device from the body lumen.

Preferably the centring means is a centring catheter and the method includes the step of moving the centring catheter from an introduction configuration in which the distal end of the centring catheter projects distally from the retrieval catheter for crossing a lesion or a treatment device and a retrieval configuration in which the centring catheter is proximal of the retrieval space for retrieving the medical device.

Preferably the retrieval catheter and the centring catheter are introduced into the body lumen over a guidewire. The centring catheter positions the guidewire centrally in the lumen away from the lumen walls.

The distal end of the retrieval catheter expands radially outwardly during retrieval of the medical device into the retrieval catheter. Thus the retrieval catheter may retrieve medical devices with a diameter greater than the relaxed diameter of the catheter. The distal end of the retrieval catheter may expand radially outwardly during retraction of the distal end of the centring catheter into the retrieval catheter. Typically the distal end of the centring catheter is deformed as the distal end of the centring catheter is retracted into the retrieval catheter.

In another embodiment the retrieval catheter has a main catheter body, the centring means is a tapered distal extension of the main body of the retrieval catheter and the tapered distal extension has an open mouth through which a medical device is retrieved.

In one case at least portion of the distal extension is movable inwardly between the introduction and retrieval configurations.

Alternatively at least portion of the distal extension is movable radially outwardly between the introduction and retrieval configurations.

In a preferred embodiment the method includes the step of flushing and/ or aspirating before retrieving the medical device into the retrieval catheter. Ideally the method includes the step of removing the centring catheter from the retrieval catheter to facilitate flushing and/or aspiration. This provides more space within the retrieval catheter for the flushing and/or aspiration.

In one embodiment the medical device is retrieved into the retrieval catheter by drawing the medical device proximally into the retrieval catheter. In an alternative embodiment the medical device is retrieved into the retrieval catheter by advancing the retrieval catheter distally relative to the medical device. The medical device may also be retrieved by a combination of drawing the medical device proximally and advancing the catheter distally.

The centring catheter may be removed from the body lumen before removing the retrieval catheter and the medical device from the body lumen.

Ideally the centring catheter structurally supports the retrieval catheter during introduction into the body lumen. This enables a very thin-walled low-profile retrieval catheter to be used.

Preferably the medical device is mounted on or engagable with a guidewire.

In one preferred embodiment the medical device is an embolic filter device.

The method for retrieving a medical device from a body lumen is particularly suitable for retrieving an embolic filter device from a blood vessel that has been deployed distal to an implanted stent in the treated blood vessel.

### Brief Description of the Drawings

The invention will be more clearly understood from the following description thereof given by way of example only, in which: -
Fig. 1 is a side view of a retrieval device according to the invention;
Fig. 2 is a perspective partially cut-away view of the retrieval device of Fig. 1, in use;
Figs. 3(a) to 3(d) are side partially cut-away views of the retrieval device of Fig. 1 in different positions of use;
Fig. 4 is a diagrammatic view of the retrieval device of Fig. 1 in use;
Fig. 5 is an enlarged cross sectional view of a distal end of the retrieval device of Fig. 4;
Fig. 6 is a side cross sectional view of a distal end detail of another retrieval device according to the invention;
Figs. 7 to 9 are schematic side views of the retrieval device of Fig. 6 in use;
Fig. 10 is a side cross sectional view of a distal end detail of another retrieval device according to the invention;
Fig. 11 is a side cross sectional view of the retrieval device of Fig. 10 in an advancement mode;
Fig. 12 is a side cross sectional view of the retrieval device of Fig. 10 in use;
Fig. 13 is a side cross sectional view of a distal end detail of yet another retrieval device according to the invention;
Figs. 14 to 17 are schematic side views of the retrieval device of Fig. 13 in use;
Fig. 18 is a side cross sectional view of a distal end detail of a further retrieval device according to the invention;
Fig. 19 is a side cross sectional view of a distal end detail of another retrieval device not according to the invention;
Fig. 20 is a perspective partially cut-away view of a detail of a catheter assembly of the invention;
Fig. 21 is a perspective partially cut-away view of a detail of another catheter assembly of the invention;
Fig. 22 is a cross sectional end view of a detail of another catheter assembly of the invention.
Fig 23 is a side, partially cross sectional view of a retrieval device according to another embodiment of the invention;
Figs 24 to 26 are views of the retrieval device of Fig 23 in use for retrieval of a distal embolic filter;
Fig 27 is a side, partially cross sectional view of another retrieval device according to the invention;
Figs 28 to 30 are views of the retrieval device of Fig 27, in use; and
Fig 31 is a perspective view of an enlarged scale of the distal tip of the retrieval device of Figs 27 to 30.

### Detailed Description

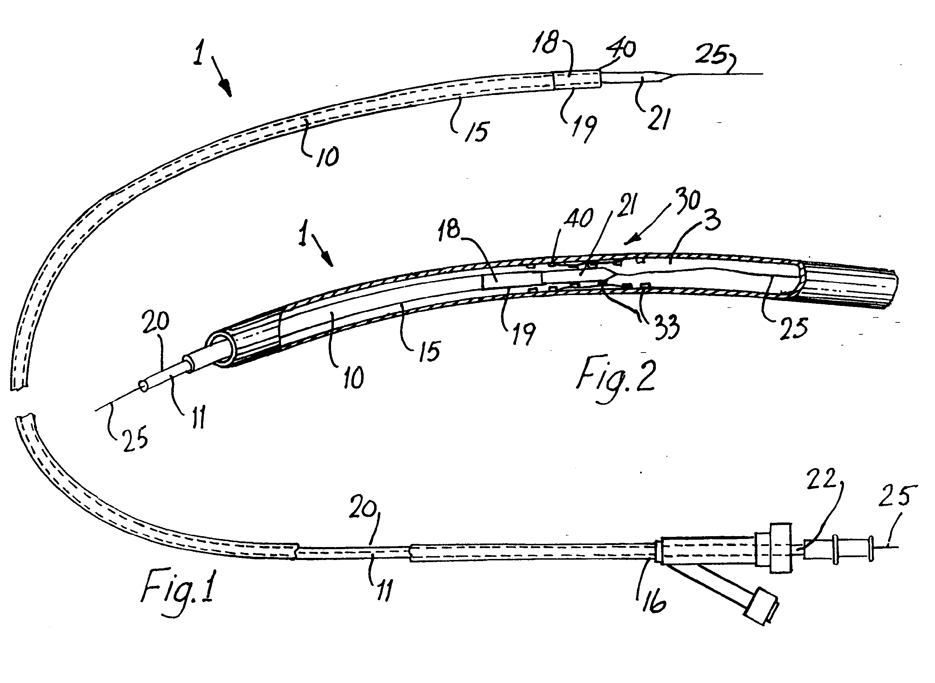

Referring to Figs. 1 to 5 there is illustrated a retrieval device according to the invention and indicated generally by the reference numeral 1. The retrieval device 1 is used for retrieval of an embolic filter 2 from a body lumen such as a vasculature 3. The filter 2 is located distally of a lesion or another treatment device such as a stent. The embolic filter 2 is of the type described in our WO 99/23976A.

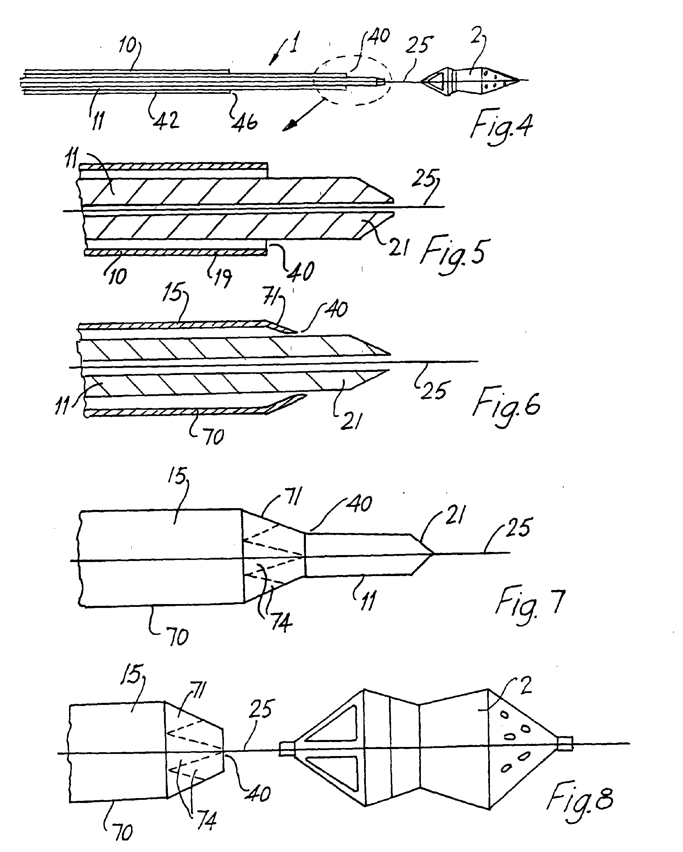

In use a guide catheter 42 (Fig. 4) extends through the body lumen 3 proximal to a stenosed region 30. The guide catheter 42 comprises an elongate tube having a proximal end and a distal end. The distal end has an open mouth 46. To retrieve the embolic filter 2 a retrieval device 1 is advanced through the guide catheter 42. The retrieval device 1 comprises a retrieval catheter 10 and a centring catheter 11.

The retrieval catheter 10 comprises an elongate flexible tube 15 having a proximal end 16 external of the lumen and fitted, for example with a Y-connector and a Tuohy Borst adapter. The retrieval catheter 10 extends through the guide catheter 42 and terminates beyond the distal end thereof. A distal end 18 of the retrieval catheter has an expansible tip 19 with an open mouth 40 to accommodate the embolic filter 2.

The centring catheter 11 comprises an elongate tube 20 which extends through the retrieval catheter tube 15 and terminates beyond the distal end 18 thereof in a soft, flexible tapered end 21. A proximal end 22 of the centring catheter 11 extends from the body lumen for external manipulation of the centring catheter 11. In this case the centring catheter tube 20 has a central bore for threading over a guidewire 25 to which the filter 2 may be mounted. The bore may extend only partially along the length of the centring catheter 11 to provide for rapid exchange. For ease of location, preferably distal end 21 of the centring catheter 11 is at least partially of, or coated with, a radiopaque material. The centring catheter 11 is of a suitable low friction material for smooth slidable travel through the retrieval catheter 10. A particularly suitable material for the centring catheter 11 would be a high density polyethylene/low density polyethylene (HDPE/LDPE) mix.

The tapered distal end 21 of the centring catheter 11, in use, projects from the open mouth 40 of the retrieval catheter 10 (Fig. 5). The soft, tapered distal end 21 allows the retrieval catheter 10 to be smoothly advanced through the vasculature 3 without becoming snagged on a partial blockage such as a region of stenosis, or on a deployed stent 33, or in stenosed regions 31, 32 adjacent to the stent 33. The taper may be straight as illustrated in Fig. 5, or may be curved in a concave manner.

To collapse the filter 2 after use and retrieve it from the body lumen 3, the retrieval catheter 10 is introduced over the guidewire 25 on which the filter 2 is mounted, in this case. With the aid of radiopaque markers, the filter 2 is urged into the retrieval catheter 10 by pulling the guidewire 25 proximally. The tip 19 of the retrieval catheter 10 is radially flexible/expansible to accommodate the embolic bulk that the filter 2 may have retained, but also has enough axial stiffness to assist the pull back of the filter 2 into the tip 19.

Alternatively the filter 2 may be retrieved into the tip 19 by advancing the retrieval catheter 10 further distally relative to the filter 2, thereby collapsing the filter 2.

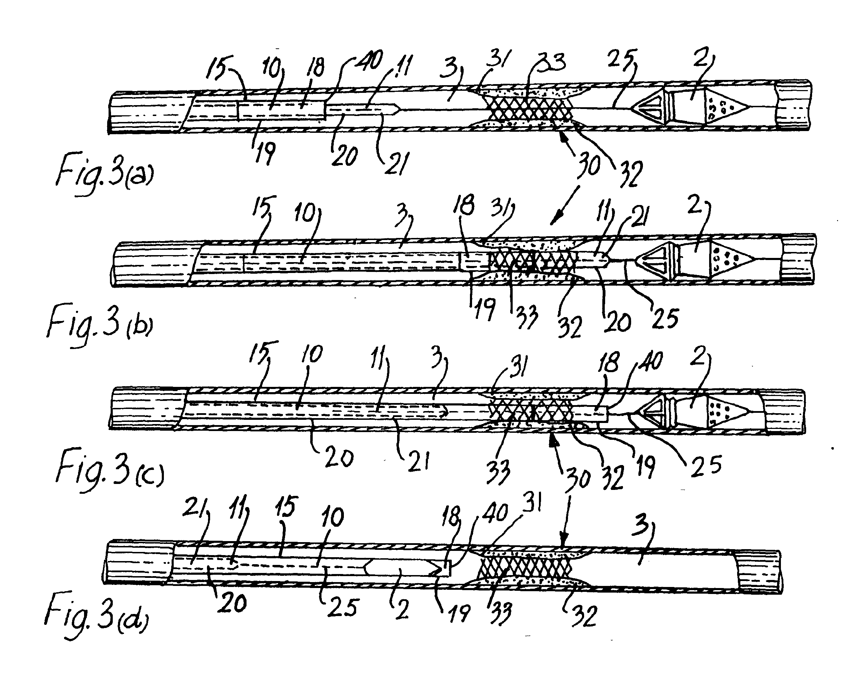

Referring in particular to Figs. 3(a) to 3(d), in an advancement mode the tapered distal end 21 of the centring catheter 11 projects from the open distal end 18 of the retrieval catheter 10 over the guidewire 25 (Fig. 3(a)). The tapered distal end 21 of the centring catheter 11 guides the retrieval device 1 through the deployed stent 33 without snagging the retrieval catheter 10 on the stent 33 or the stenosed region 30 (Fig. 3(b)). When the retrieval catheter 10 has been advanced so that the distal end 21 of the centring catheter 11 is in a position adjacent the proximal end of the filter 2, the tapered end 21 of the centring catheter 11 is retracted proximally into the retrieval catheter 10, exposing the open mouth 40 of the retrieval catheter 10 (Fig. 3(c)). The filter 2 is then collapsed and retrieved into the expansible tip 19 of the retrieval catheter 10. The retrieval catheter 10 with the retrieved filter 2 in place is then withdrawn through the lumen 3 (Fig. 3(d)).

Figs. 6 to 9 show another embodiment of the retrieval catheter, which is similar to the retrieval catheter 10 of Figs. 1 to 5. In this case a retrieval catheter 70 comprises an elongate tube 15 with a tapered expandable tip 71 at the distal end of the tube 15. An open mouth 40 is provided at the distal end of the tip 71 to receive the retrieved embolic filter 2 within the tip 71. The distal end of the tube 15 terminates in a plurality of splined fingers 74, over which the tip 71 is attached.

The tip 71 is of a flexible elastic polymeric material, for example polyurethane or a flexible grade of PEBA and the tube 15 is of a stiff material, for example a stiff grade of PEBAX. The tip 71 may be attached over the fingers 74 of the tube 15 by welding or by overmoulding.

Fig. 7 illustrates the advancement mode for the retrieval device, in which the distal end 21 of the centring catheter 11 projects from the open mouth 40 of the tip 71. The filter 2 is retrieved into the tip 71 by pulling the guidewire 25 proximally.

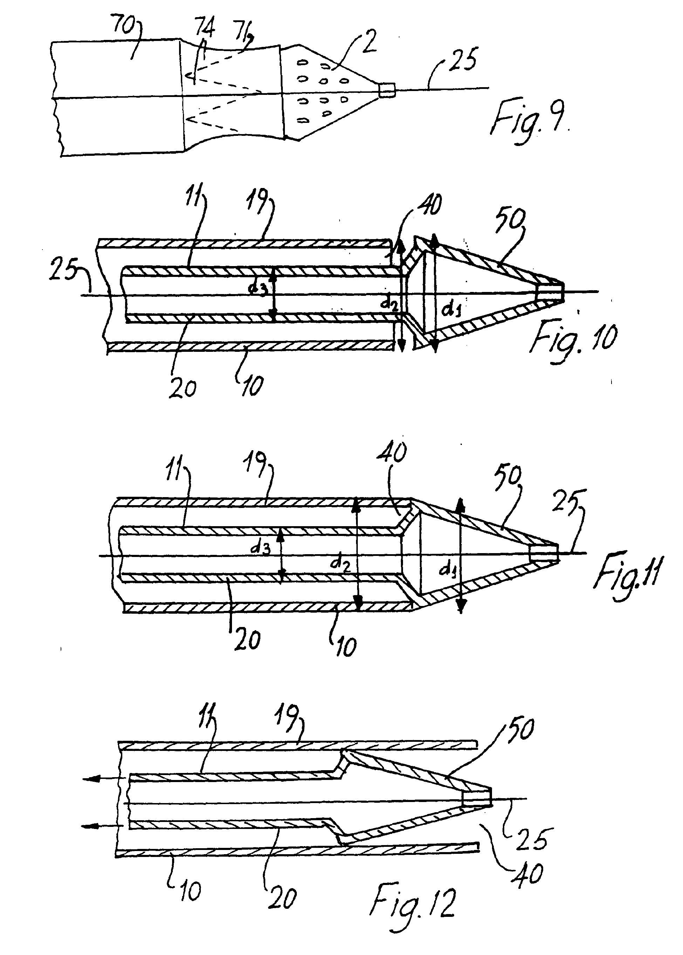

The tapered tip 71 expands outwardly as the filter 2 is retracted into the tip 71 to fully accommodate the retained embolic bulk within the filter 2, as illustrated in Figs. 8 and 9.

The filter 2 may alternatively be retrieved into the tip 71 by advancing the retrieval catheter 70 distally relative to the filter 2.

The tip 71 is sufficiently expansible to facilitate retrieval of the filter 2, even with a large embolic load retained within the filter 2.

The tapered profile of the expansible tip 71 provides a smooth, step-free transition between the centring catheter 11 and the retrieval catheter 70. This prevents hang-up of the open mouth 40 of the retrieval catheter 70 or causing further vessel trauma, as the retrieval device is advanced through the vasculature 3, in particular as the retrieval device crosses the stent 33 and stenosed regions 31, 32.

Referring now to Figs. 10 to 12 in another embodiment of the invention, the distal end of the centring catheter tube 20 is in the shape of an arrowhead 50. The largest outer diameter d₁ of the distal end 50 of the centring catheter 11 is equal to the outer diameter d₂ of the retrieval catheter tip 19 at the open mouth 40. This ensures that there is a smooth, step free crossing profile between the centring catheter distal end 50 and the retrieval catheter tip 19 in the advancement mode of Fig. 11. Also the distal end 50 of the centring catheter tube 20 sealingly engages the retrieval catheter tip 19 in the advancement mode. This enables a centring catheter tube 20 with a diameter d₃ which is smaller than d₁ to be used, thus providing more lumen space between the centring catheter tube 20 and the retrieval catheter 10 to facilitate flushing and/or aspiration.

The distal end 50 of the centring catheter tube 20 is of a soft, flexible material, and is hollow. The distal end 50 may be deformed into a smaller diameter by pulling the centring catheter 11 proximally into the retrieval catheter 10 as illustrated in Fig. 12. This enables the smooth pull back of the centring catheter 11 into the retrieval catheter 10 prior to retrieval of the filter 2 into the tip 19. Pull back of the centring catheter 11 is further assisted by the axial stiffness of the tip 19 of the retrieval catheter 10.

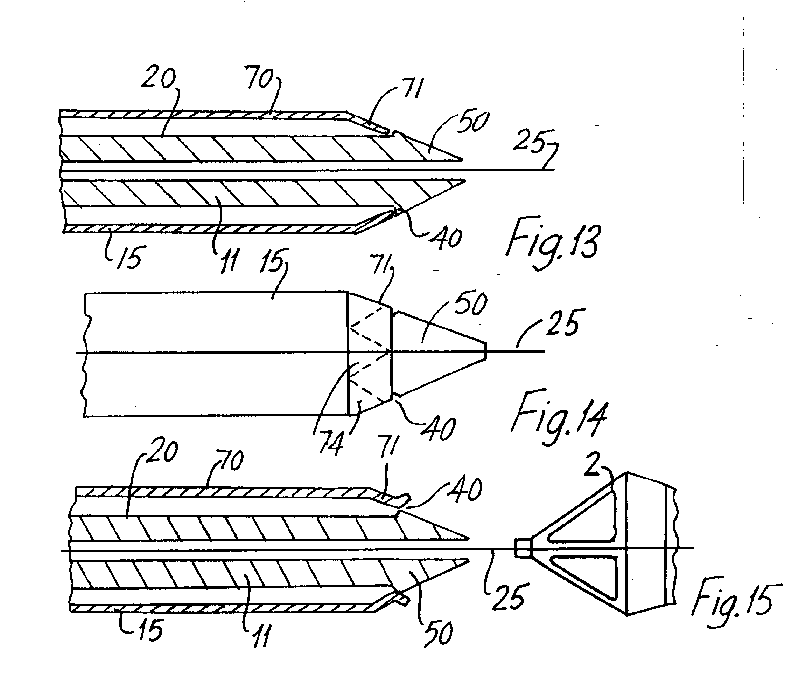

A further embodiment of the invention is illustrated in Figs. 13 to 17, in which the tapered distal tip 71 of the retrieval catheter 70 tapers towards the arrowhead-shaped distal end 50 of the centring catheter 11. Thus, in the advancement mode the retrieval device has a smooth, step-free crossing profile, as illustrated in Figs. 13 and 14.

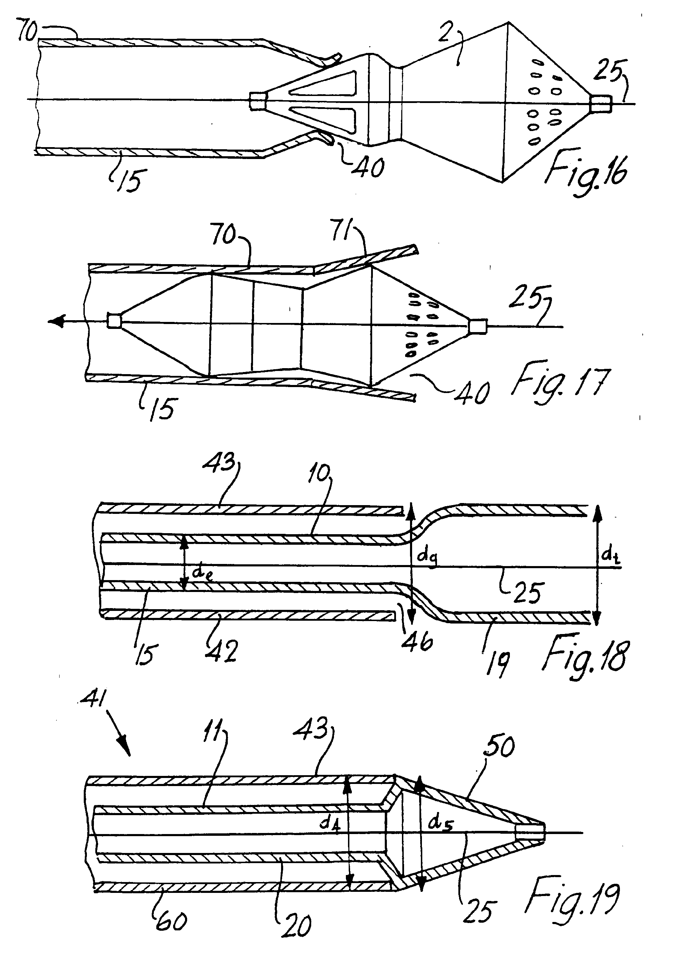

The tip 71 is radially expansible to enable retraction of the centring catheter tip 50 through the open mouth 40 proximally into the retrieval catheter 70 before retrieval of the filter 2 (Fig. 15), and then to enable retraction of the filter 2 together with the retained embolic load by pulling the guidewire 25 proximally (Figs. 16 and 17).

The filter 2 may alternatively be retrieved into the tip 71 by advancing the retrieval catheter 70 distally relative to the filter 2 and/or centring catheter 11.

Referring now to Fig. 18 in another embodiment of the invention the diameter of the retrieval catheter 10 varies along its longitudinal length. In this case the diameter dₜ of the tip 19 of the retrieval catheter 10 is greater than the diameter dₑ of the remaining length of the retrieval catheter tube 15. The diameter dₜ of the tip 19 of the retrieval catheter 10 is equal to or greater than the diameter dg of the guide catheter 42. This allows a retrieval catheter tube 15 of smaller diameter to be used, thus providing more lumen space between the retrieval catheter tube 15 and the guide catheter 42. This extra lumen space enables the injection of contrast media and the like through the guide catheter 42.

Referring to Fig. 19 there is illustrated another retrieval device 41 according to the invention, which is similar to the retrieval device 1 and like parts are assigned the same reference numerals. In this case the retrieval catheter 41 comprises a guide catheter 60 and a centring catheter 11, and a separate retrieval catheter is not required. The guide catheter 60 is of sufficiently small diameter to allow it to be advanced to the site of the stenosis 30, and the distal end 50 of the centring catheter 11 in the shape of an arrowhead is sized to match the outer diameter of the guide catheter 60. The largest outer diameter d₅ of the distal end 50 is equal to the outer diameter d₄ of the guide catheter 60. This ensures a smooth, step-free transition between the centring catheter 11 and the guide catheter 60.

The invention provides a retrieval system, which is simple to operate and yet provides snag-free manipulation of a retrieval catheter to a retrieval site. A smooth, step-free crossing profile is provided with a tapered transition to enable the retrieval catheter to pass through the diseased vessel and cross the stent without gouging the vessel, without catching on or dislodging the stent and without scraping or dislodging embolic material from the wall of the vessel or stent.

The centring catheter 11 also provides support to the retrieval catheter assembly, greatly enhancing the integrity and kink resistance of the system, so that an ultra low profile, thin wall retrieval catheter 10 can be used without compromising the integrity of the system. This provides a very large lumen within the tip 19 of the retrieval catheter 10 to facilitate the retrieval of large filters with large volumes of captured embolic material. In one embodiment of the invention the centring catheter has an inner diameter of 0.76 mm and an outer diameter of 1.63mm, and the retrieval catheter has an inner diameter of 1.78 mm and an outer diameter of 2.08 mm. These dimensions are given as examples only and are by no means essential to the invention. It will be appreciate that other diameters may also be used to obtain a retrieval system with structural integrity and a large retrieval lumen.

The centring catheter 11 also maintains the guidewire 25 positioned towards the centre of the lumen 3 away from the walls of the lumen 3. This minimises the contact between the guidewire 25 and the walls of the lumen 3 to avoid further vessel trauma or release of embolic material from the vessel wall.

The invention has been described in relation to a carotid angioplasty procedure with an intravascular filter being placed distally to capture any emboli being released during the procedure. However, it may be applied to other medical procedures such as angioplasty and stenting of surgically implanted Saphenous Vein grafts that have stenosed, or primary treatment of renal artery stenoses. Indeed, the invention is applicable to the retrieval of embolic protection devices to protect patients during any vascular intervention.

Preferably the retrieval device 1 includes means to facilitate flushing and/or aspiration. In one embodiment of the invention, the centring catheter 11 may be withdrawn fully from the retrieval catheter 10, to facilitate flushing and/or aspiration.

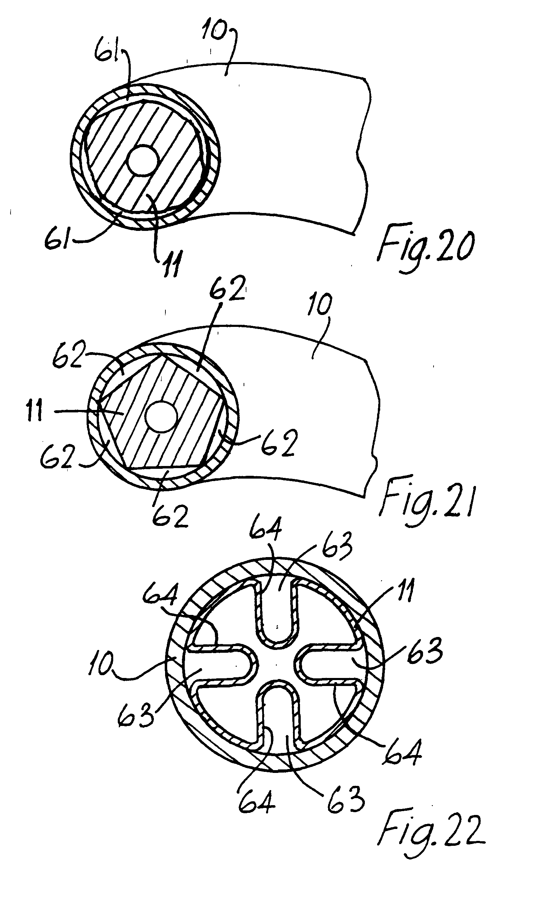

Referring to Figs. 20 to 22 there are illustrated alternative centring catheters 11 with non-circular cross sections to facilitate flushing and/or aspiration without requiring the centring catheter 11 to be removed from the retrieval catheter 10. The non-circular cross section also reduces the area of contact between the centring catheter 11 and the retrieval catheter 10, thereby reducing the frictional force acting between the outer surface of the centring catheter tube 20 and the inner surface of the retrieval catheter tube 15.

Referring in particular to Fig. 20 in this case the centring catheter 11 is of a generally oval cross section. This creates defined passageways 61 between the retrieval catheter 10 and the centring catheter 11.

In the arrangement illustrated in Fig. 21 there are five contact points between the centring catheter 11 and the retrieval catheter 10, and again defined passageways 62 are created.

The fluted arrangement of Fig. 22 is preferred because defined passageways 63 are created between the flutes 64 while ensuring a large lumen space between the centring catheter 11 and the retrieval catheter 10.

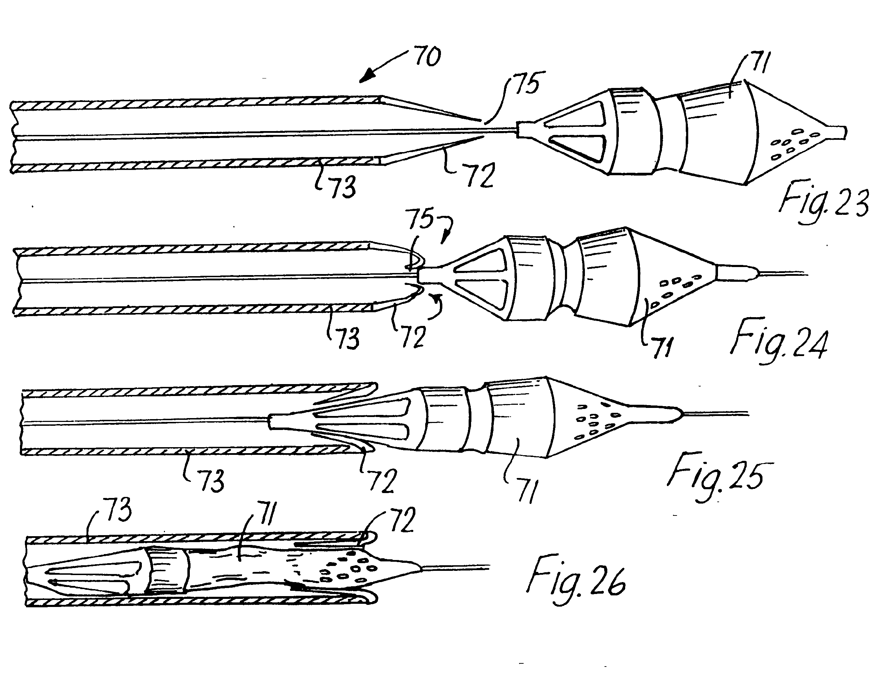

Referring to Figs 23 to 26 there is illustrated another retrieval device 70 for retrieving a medical device such as a distal embolic filter 71 located distally of a lesion or a treatment device. The retrieval device 70 has a centring means in the form of a tapered distal extension 72 of a main body of a retrieval catheter 73. The tapered distal extension 72 has an open mouth 75 through which the filter 71 is retrieved.

In this case at least portion of the distal extension 72 is movable inwardly in the direction of the arrows in Fig 24 between an introduction configuration as illustrated in Fig 23 in which the retrieval device crosses a lesion or another treatment device such as a stent and is advanced to the filter 71 . As the filter 71 is pulled back and collapses into the retrieval catheter 23 the distal extension 72 is forced inwardly into the retrieval catheter into the final retrieval configuration illustrated in Fig 26 far withdrawn from the body lumen.

The distal extension 72 may be of the same material as that of the main body 73 of the retrieval catheter but with substantially reduced wall thickness. Alternatively or additionally the extension may be of a resilient elastomeric material such as a soft polyurethane, PEBAX or PTFE.

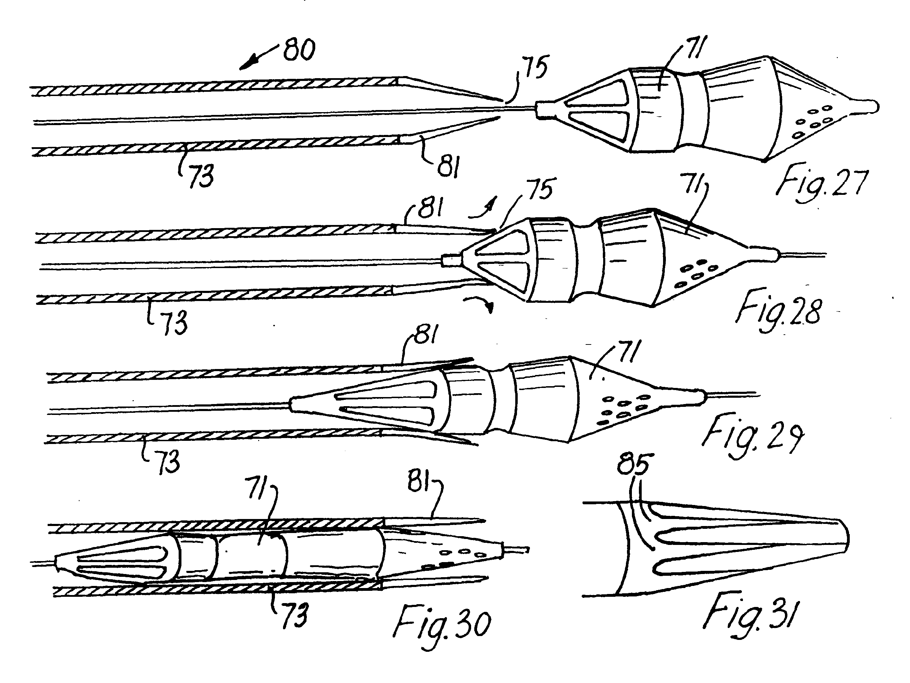

Referring to Figs 27 to 31 there is illustrated another retrieval device 80 similar to the device of Figs 23 to 26 and like parts are assigned the same reference numerals. In this case the distal extension 81 is movable radially outwardly between the introduction and retrieval configurations. The tapered distal extension 81 may be similar to the arrangement of Figs 6 to 9. Ideally the extension 81 has a number of tapered reinforcing legs 85 covered in a softer elastic material forming a conical but expansible pod for the reception of the filter 70. The rigid reinforcing legs 85 ensure the tip of that the distal extension is not inverted and assist in centring the guidewire.

The invention is not limited to the embodiments hereinbefore described, which may be varied in detail.

## Claims

1. An embolic filter retrieval device for retrieving an embolic filter (2) from a body lumen, through a lesion or a treatment device proximal of the filter (2), the retrieval device comprising: -
a retrieval catheter (10) including an elongate tube (15) having a proximal end (16) and a distal end (18), the retrieval catheter (10) defining a retrieval space, into which an embolic filter (2) is at least partially retrieved, at the distal end (8) of the elongate tube (15);
a centring catheter (11) having a distal end (21), the centring catheter (11) being mounted in the retrieval catheter (10) for movement between an introduction configuration, for introduction of the retrieval catheter (10) through a lesion or a treatment device to an embolic filter (2) positioned outside the retrieval space of the retrieval catheter (10), in which the distal end (21) of the centring catheter (11) projects distally from the distal end (18) of the retrieval catheter (10), and a retrieval configuration in which the distal end (21) of the centring catheter (11) is proximal of the retrieval space of the retrieval catheter (10) and the embolic filter (2) is at least partially retrieved into the retrieval space of the retrieval catheter (10);
a guidewire (25) extending at least in part through the centring catheter (10), the guidewire (25) having a proximal end and a distal end;
said embolic filter (2) mounted on or engagable with the guidewire (25) proximate the distal end of the guidewire (25);
**characterised in that** the retrieval catheter (10) has a radially expansible axially stiff tip (19) at the distal end to accommodate retrieval of the embolic filter (2) into the retrieval space.

2. A retrieval device as claimed in claim 1 wherein the centring catheter (11) has an internal bore extending at least partially along its length for threading the retrieval device over a guidewire (25).

3. A retrieval device as claimed in claim 1 or 2 wherein the centring catheter (11) is slidably movable in the retrieval catheter (10) from the introduction configuration to the retracted retrieval configuration.

4. A retrieval device as claimed in any of claims 1 to 3 wherein the centring catheter (11) is removable from the retrieval catheter (10).

5. A retrieval device as claimed in any of claims 1 to 4 wherein a proximal end (22) of the centring catheter (11) extends proximally from the proximal end (16) of the retrieval catheter (10) for external manipulation of the centring catheter (11) relative to the retrieval catheter (10).

6. A retrieval device as claimed in any of claims 1 to 5 wherein the distal end. (21) of the centring catheter (11) is tapered distally inwardly to guide the open mouth through the body lumen.

7. A retrieval device as claimed in any of claims 1 to 5 wherein the distal end (21) of the centring catheter (11) is shaped to provide a smooth transition between the distal end (21) of the centring catheter (11) and the distal end (18) of the retrieval catheter (10).

8. A retrieval device as claimed in claim 7 wherein the distal end (50) of the centring catheter (17) is of arrowhead shape.

9. A retrieval device as claimed in claim 7 or 8 wherein the distal end (50) of the centring catheter (11) is flexible.

10. A retrieval device as claimed in any of claims 7 to 9 wherein the distal end (50) of the centring catheter (11) is sealably engagable to the distal end of the retrieval catheter (10).

11. A retrieval device as claimed in any preceding claim wherein the distal end (21) of the centring catheter (11) is at least partially radiopaque.

12. A retrieval device as claimed in any preceding claim wherein the centring catheter (11) is of or coated with a material having a low coefficient of friction.

13. A retrieval device as claimed in any preceding claim wherein the diameter of the retrieval catheter (10) varies along its length.

14. A retrieval device as claimed in any preceding claim wherein the distal end (71) of the retrieval catheter (70) is tapered distally inwardly to provide a smooth crossing profile for the retrieval device.

15. A retrieval device as claimed in any of claims 1 to 14 wherein the guidewire (25) is pullable proximally for retrieval of at least part of the embolic filter (2) into the retrieval space.

16. A retrieval device as claimed in any of claims 1 to 15 wherein the inner surface of the retrieval catheter (10) and/or the outer surface of the centring catheter (11) is of non-circular profile over at least portion of the length thereof.

17. A retrieval device as claimed in claim 16 wherein the outer surface of the centring catheter (11) is non-circular.

18. A retrieval device as claimed in claim 16 or 17 wherein the outer surface of the centring catheter (11) is of oval shape.

19. A retrieval device as claimed in any of claims 16 to 18 wherein the outer surface of the centring catheter (11) is shaped to define a number of separate areas of contact with the retrieval catheter (10).

20. A retrieval device as claimed in claim 19 wherein the outer surface of the centring catheter (11) is of fluted shape.

21. A device as claimed in any of claims 1 to 20 wherein the distal end of the elongate tube (15) terminates in a plurality of splined fingers (74) over which the tip (71) is attached.

22. A device as claimed in any of claims 1 to 21 wherein the expansible tip (19, 71) is of a flexible polymeric material.

23. A device as claimed in claim 22 wherein the tube (15) is of a material which is stiff with respect to the flexible polymeric material.

## Patentansprüche

1. Emboliefilter-Bergungsvorrichtung zum Bergen eines Emboliefilters (2) aus einem Körperlumen durch eine Läsion oder eine Behandlungseinrichtung proximal von dem Filter (2), wobei die Bergungsvorrichtung Folgendes umfasst:
einen Bergungskatheter (10), der ein längliches Röhrchen (15) mit einem proximalen Ende (16) und einem distalen Ende (18) aufweist, wobei der Bergungskatheter (10) einen Bergungsraum an dem distalen Ende (8) des länglichen Röhrchens (15) begrenzt, in den ein Emboliefilter (2) wenigstens teilweise geborgen wird;
einen Zentrierkatheter (11) mit einem distalen Ende (21), wobei der Zentrierkatheter (11) in dem Bergungskatheter (10) zur Bewegung zwischen einer Einführungskonfiguration, zur Einführung des Bergungskatheters (10) durch eine Läsion oder eine Behandlungseinrichtung zu einem Emboliefilter (2), der außerhalb des Bergungsraums des Bergungskatheters (10) positioniert ist, in der das distale Ende (21) des Zentrierkatheters (11) distal von dem distalen Ende (18) des Bergungskatheters (10) vorsteht, und einer Bergungskonfiguration angebracht ist, in der sich das distale Ende (21) des Zentrierkatheters (11) proximal von dem Bergungsraum des Bergungskatheters (10) befindet und der Emboliefilter (2) wenigstens teilweise in den Bergungsraum des Bergungskatheters (10) geborgen wird;
einen Führungsdraht (25), der sich wenigstens teilweise durch den Zentrierkatheter (10) erstreckt, wobei der Führungsdraht (25) ein proximales Ende und ein distales Ende aufweist;
den Emboliefilter (2), der an dem Führungsdraht (25) nahe dem distalen Ende des Führungsdrahts (25) angebracht ist oder mit demselben in Eingriff gebracht werden kann;
**dadurch gekennzeichnet, dass** der Bergungskatheter (10) eine radial ausfahrbare, axial steife Spitze (19) an dem distalen Ende aufweist, um Bergung des Emboliefilters (2) in den Bergungsraum anzupassen.

2. Bergungsvorrichtung nach Anspruch 1, bei der der Zentrierkatheter (11) eine Innenbohrung aufweist, die sich wenigstens teilweise entlang seiner Länge zum Einfädeln der Bergungsvorrichtung über einen Führungsdraht (25) erstreckt.

3. Bergungsvorrichtung nach Anspruch 1 oder 2, bei der der Zentrierkatheter (11) gleitend in dem Bergungskatheter (10) von der Einführungskonfiguration in die eingezogene Bergungskonfiguration bewegt werden kann.

4. Bergungsvorrichtung nach einem der Ansprüche 1 bis 3, bei der der Zentrierkatheter (11) aus dem Bergungskatheter (10) entfernt werden kann.

5. Bergungsvorrichtung nach einem der Ansprüche 1 bis 4, bei der sich ein proximales Ende (22) des Zentrierkatheters (11) proximal von dem proximalen Ende (16) des Bergungskatheters (10) zur externen Handhabung des Zentrierkatheters (11) in Bezug zu dem Bergungskatheter (10) erstreckt.

6. Bergungsvorrichtung nach einem der Ansprüche 1 bis 5, bei der sich das distale Ende (21) des Zentrierkatheters (11) distal nach innen verjüngt, um das offene Mundstück durch das Körperlumen zu führen.

7. Bergungsvorrichtung nach einem der Ansprüche 1 bis 5, bei der das distale Ende (21) des Zentrierkatheters (11) geformt ist, um einen sanften Übergang zwischen dem distalen Ende (21) des Zentrierkatheters (11) und dem distalen Ende (18) des Bergungskatheters (10) bereitzustellen.

8. Bergungsvorrichtung nach Anspruch 7, bei der das distale Ende (50) des Zentrierkatheters (11) pfeilspitzenförmig ist.

9. Bergungsvorrichtung nach Anspruch 7 oder 8, bei der das distale Ende (50) des Zentrierkatheters (11) flexible ist.

10. Bergungsvorrichtung nach einem der Ansprüche 7 bis 9, bei der das distale Ende (50) des Zentrierkatheters (11) abdichtend mit dem distalen Ende des Bergungskatheters (10) in Eingriff gebracht werden kann.

11. Bergungsvorrichtung nach einem vorhergehenden Anspruch, bei der das distale Ende (21) des Zentrierkatheters (11) mindestens teilweise strahlenundurchlässig ist.

12. Bergungsvorrichtung nach einem vorhergehenden Anspruch, bei der der Zentrierkatheter (11) aus einem Material mit einem niedrigen Reibungskoeffizienten besteht oder mit diesem beschichtet ist.

13. Bergungsvorrichtung nach einem vorhergehenden Anspruch, bei dem der Durchmesser des Bergungskatheters (10) über seine Länge variiert.

14. Bergungsvorrichtung nach einem vorhergehenden Anspruch, bei der sich das distale Ende (71) des Bergungskatheters (70) distal nach innen verjüngt, um ein glattes Querprofil für die Bergungsvorrichtung bereitzustellen.

15. Bergungsvorrichtung nach einem der Ansprüche 1 bis 14, bei der der Führungsdraht (25) zum Bergen wenigstens eines Teils des Emboliefilters (2) in den Bergungsraum proximal gezogen werden kann.

16. Bergungsvorrichtung nach einem der Ansprüche 1 bis 15, bei der die Innenfläche des Bergungskatheters (10) und/oder die Außenfläche des Zentrierkatheters (11) über wenigstens einen Teil der Länge desselben ein nicht kreisförmiges Profil hat.

17. Bergungsvorrichtung nach Anspruch 16, bei der die Außenfläche des Zentrierkatheters (11) nicht kreisförmig ist.

18. Bergungsvorrichtung nach Anspruch 16 oder 17, bei der die Außenfläche des Zentrierkatheters (11) eine ovale Form hat.

19. Bergungsvorrichtung nach einem der Ansprüche 16 bis 18, bei der die Außenfläche des Zentrierkatheters (11) so geformt ist, um eine Anzahl getrennter Berührungsflächen mit dem Bergungskatheter (10) zu definieren.

20. Bergungsvorrichtung nach Anspruch 19, bei der die Außenfläche des Zentrierkatheters (11) eine geriffelte Form hat.

21. Vorrichtung nach einem der Ansprüche 1 bis 20, bei der das distale Ende des länglichen Rohrs (15) in einer Mehrzahl von Keilfingem (74) endet, über denen die Spitze (71) befestigt wird.

22. Vorrichtung nach einem der Ansprüche 1 bis 21, bei der die ausfahrbare Spitze (19, 71) aus einem flexiblen Polymermaterial besteht.

23. Vorrichtung nach Anspruch 22, bei der das Röhrchen (15) aus einem Material besteht, das in Bezug zu dem flexiblen Polymermaterial steif ist.

## Revendications

1. Dispositif d'extraction d'un filtre embolique pour extraire un filtre embolique (2) d'une lumière d'un corps, à travers une lésion ou un dispositif de traitement proximal au filtre (2), le dispositif d'extraction comprenant :
un cathéter d'extraction (10) comprenant un tube allongé (15) muni d'une extrémité proximale (16) et une extrémité distale (18), le cathéter d'extraction (10) délimitant un espace d'extraction, dans lequel un filtre embolique (2) est extrait au moins en partie, à l'extrémité distale (8) du tube allongé (15) ;
un cathéter de centrage (11) doté d'une extrémité distale (21), le cathéter de centrage (11) étant monté dans le cathéter d'extraction (10) pour se déplacer entre une configuration d'introduction, pour l'introduction du cathéter d'extraction (10) à travers une lésion ou un dispositif de traitement jusqu'à un filtre embolique (2) positionné hors de l'espace d'extraction du cathéter d'extraction (10), dans lequel l'extrémité distale (21) du cathéter de centrage (11) se projette en direction distale depuis l'extrémité distale (18) du cathéter d'extraction (10), et une configuration d'extraction dans laquelle l'extrémité distale (21) du cathéter de centrage (11) est proximale à l'espace d'extraction du cathéter d'extraction (10) et le filtre embolique (2) est au moins partiellement extrait dans l'espace d'extraction du cathéter d'extraction (10) ;
un fil-guide (25) s'étendant au moins partiellement à travers le cathéter de centrage (10), le fil-guide (25) étant doté d'une extrémité proximale et d'une extrémité distale ;
ledit filtre embolique (2) étant monté sur ou pouvant s'engager avec le fil-guide (25) à proximité de l'extrémité distale du fil-guide (25) ;
**caractérisé par le fait que** le cathéter d'extraction (10) a un bout radialement extensible et axialement rigide (19) à l'extrémité distale pour recevoir l'extraction du filtre embolique (2) dans l'espace d'extraction.

2. Dispositif d'extraction conforme à la revendication 1, où le cathéter de centrage (11) est doté d'une âme interne s'étendant au moins partiellement sur sa longueur pour enfiler le dispositif d'extraction sur un fil-guide (25).

3. Dispositif d'extraction conforme à la revendication 1 ou 2, où le cathéter de centrage (11) peut se déplacer en glissant dans le cathéter d'extraction (10) depuis la configuration d'introduction jusqu'à la configuration d'extraction rétractée.

4. Dispositif d'extraction conforme à une quelconque des revendications 1 à 3, où le cathéter de centrage (11) est extractible du cathéter d'extraction (10).

5. Dispositif d'extraction conforme à une quelconque des revendications 1 à 4, où une extrémité proximale (22) du cathéter de centrage (11) s'étend en direction proximale depuis l'extrémité proximale (16) du cathéter d'extraction (10) pour permettre la manipulation externe du cathéter de centrage (11) par rapport au cathéter d'extraction (10).

6. Dispositif d'extraction conforme à une quelconque des revendications 1 à 5, où l'extrémité distale (21) du cathéter de centrage (11) est effilée vers l'intérieur en direction distale pour guider la bouche ouverte à travers la lumière du corps.

7. Dispositif d'extraction conforme à une quelconque des revendications 1 à 5, où l'extrémité distale (21) du cathéter de centrage (11) est dotée d'une forme apte à fournir une transition lisse entre l'extrémité distale (21) du cathéter de centrage (11) et l'extrémité distale (18) du cathéter d'extraction (10).

8. Dispositif d'extraction conforme à la revendication 7, où l'extrémité distale (50) du cathéter de centrage (11) est en forme de pointe de flèche.

9. Dispositif d'extraction conforme à la revendication 7 ou 8, où l'extrémité distale (50) du cathéter de centrage (11) est flexible.

10. Dispositif d'extraction conforme à une quelconque des revendications 7 à 9, où l'extrémité distale (50) du cathéter de centrage (11) peut s'engager de manière hermétique avec l'extrémité distale du cathéter d'extraction (10).

11. Dispositif d'extraction conforme à une quelconque des revendications précédentes, où l'extrémité distale (21) du cathéter de centrage (11) est au moins partiellement radiopaque.

12. Dispositif d'extraction conforme à une quelconque des revendications précédentes, où le cathéter de centrage (21) est fait en ou revêtu d'une matière dotée d'un faible coefficient de friction.

13. Dispositif d'extraction conforme à une quelconque des revendications précédentes, où le diamètre du cathéter d'extraction (10) varie sur sa longueur.

14. Dispositif d'extraction conforme à une quelconque des revendications précédentes, où l'extrémité distale (71) du cathéter d'extraction (70) est effilée en direction distale vers l'intérieur pour fournir un profil de passage lisse au dispositif d'extraction.

15. Dispositif d'extraction conforme à une quelconque des revendications 1 à 14, où le fil-guide (25) peut être tiré en direction proximale pour extraire au moins une partie du filtre embolique (2) dans l'espace d'extraction.

16. Dispositif d'extraction conforme à une quelconque des revendications 1 à 15, où la surface intérieure du cathéter d'extraction (10) et/ou la surface extérieure du cathéter de centrage (11) ont un profil non circulaire sur au moins une partie de leur longueur.

17. Dispositif d'extraction conforme à la revendication 16, où la surface extérieure du cathéter de centrage (11) est non-circulaire.

18. Dispositif d'extraction conforme à la revendication 16 ou 17, où la surface extérieure du cathéter de centrage (11) est de forme ovale.

19. Dispositif d'extraction conforme à une quelconque des revendications 16 à 18, où la surface extérieure du cathéter de centrage (11) est dotée d'une forme apte à délimiter un certain nombre de zones séparées de contact avec le cathéter d'extraction (10).

20. Dispositif d'extraction conforme à la revendication 19, où la surface extérieure du cathéter de centrage (11) est de forme nervurée.

21. Dispositif conforme à une quelconque des revendications 1 à 20, où l'extrémité distale du tube allongé (15) se termine en une pluralité de doigts cannelés (74) au-dessus desquels le bout (71) est attaché.

22. Dispositif conforme à une quelconque des revendications 1 à 21, où le bout extensible (19, 71) est en polymère flexible.

23. Dispositif conforme à la revendication 22, où le tube (15) est en une matière rigide par rapport au polymère flexible.
